# EUROPEAN PATENT APPLICATION

(11) **EP 3 308 697 A1**
(43) Date of publication of application: **18.04.2018**
(21) Application number: 16306358.9
(22) Date of filing: 14.10.2016
(51) Int. Cl.: A61B 5/00, A61B 5/07, G01D 5/06

(54) **PROBE DEVICE AND METHOD OF OPERATING A PROBE DEVICE**

(71) Applicant: Alcatel Lucent, 91620 Nozay (FR)
(72) Inventor: WÜNSTEL, Klaus, 70435 Stuttgart (DE); BANNIZA, Thomas-Rolf, 70435 Stuttgart (DE)
(74) Representative: DREISS Patentanwälte PartG mbB

(57) **Abstract**

A probe device (100; 100a; 100b; 100c) for use in the human and/or animal body comprises a housing (110), at least one magnetic element (120) which is rotatably arranged within said housing (110), an induction coil (130) that is magnetically coupled with said magnetic element (120), and a control unit (140) configured to process an input signal (s1) characterizing a voltage induced in said induction coil (130).

## Description

### Field of the invention

The disclosure relates to a probe device for use in the human and/or animal body. The disclosure further relates to a method of operating such probe device and to a system comprising at least one probe device of the aforementioned type.

### Background

Conventional probe devices may e.g. be introduced into a human or animal body, e.g. into the gastro-intestinal (GI) tract of the human or the animal to measure certain physical parameters. Disadvantageously, the conventional devices use radio frequency, RF, signals for communication with a device external to the body, which requires comparatively large antennas for transmitting the RF signals. Further disadvantageously, the conventional probes are battery-powered, i.e. comprising a comparatively large installation space for the battery, while having a rather limited operating time. These factors prevent further miniaturization of the conventional probe devices and limit the usability to few fields of application.

### Summary

In view of this, some embodiments provide an improved probe device of the aforementioned type which is more flexible in use and which does not suffer from the abovementioned limitations of prior art.

Regarding the probe device of the abovementioned type, the probe device comprises a housing at least one magnetic element which is rotatably arranged within said housing, an induction coil that is magnetically coupled with said magnetic element, and a control unit configured to process an input signal characterizing a voltage induced in said induction coil. This configuration advantageously enables efficient communication and data exchange with the probe device without requiring spacious antennas for RF signals. Rather, an induction voltage induced into the induction coil by the rotatable magnetic element may be evaluated for receiving information, wherein said magnetic element may be driven by a magnetic field provided for by an external device. The exchange of information between the probe device or a component thereof and an external device will be explained in detail further below.

In the present context, the magnetic coupling between the induction coil and the magnetic element of the probe device means that said induction coil is configured and arranged relatively to said magnetic element such that at least a part of the magnetic flux of the magnetic element may at least temporarily (e.g., depending on an angular position of the rotatably arranged magnetic element) pass through said induction coil.

Further, the principle according to the embodiments enables to supply the probe device with energy from an external source, e.g. by driving a rotating movement of the magnetic element using magnetic fields generated and/or controlled by said external source. According to some embodiments, the energy of the rotational movement of the magnetic element may directly be used within the probe device, e.g. to drive an actuator provided within said probe device. According to other embodiments, the built-in induction coil of the probe device and an induction voltage induced therein during (an externally driven) rotational movement of the magnetic element may be used as a local electrical energy supply within the probe device.

According to some embodiments, the probe device does not necessarily require a local storage medium for electric energy such as a battery or the like, as energy may be provided from the external source just in time, e.g. when required. However, according to some other embodiments, a local electric energy storage medium such as a battery and/or a capacitor may be provided. If capacitors are considered according to some embodiments, double-layer capacitors ("ultracaps") are particularly preferred.

Also, the magnetic fields generated and/or controlled by said external source are not substantially attenuated by the human or animal body so that an efficient energy transfer (and also communication) is enabled between the external source and the probe device according to the embodiments, even if the probe device is positioned deeply within body tissue.

Although the probe device according to the embodiments is particularly well-suited for use in the human or animal body, according to further embodiments, it may also be used in other environments such as e.g. (biochemical) reactors, pipes, or generally any other target system where measurements are to be made and/or where agents are to be deployed, especially if a direct mechanical contact to said probe device is at least temporarily impossible. Also, as the principle according to the embodiments relies on magnetic fields to drive the rotatable magnetic element of the probe device, energy transfer from an external device to said probe device is nearly always possible, except with situations where a strong magnetic shielding is applied around the probe device.

According to an embodiment, said magnetic element comprises at least one bar magnet, wherein preferably a maximum length dimension of said bar magnet is smaller than about 15 millimeter (mm), preferably smaller than about 1.5 mm, which enables a particularly small configuration. For example, said bar magnet may substantially comprise cuboid shape with a width a, a length b, and a depth c, wherein e.g. b > a and b > c, and wherein said length b e.g. corresponds to the abovementioned maximum length dimension, wherein preferably b < 15 mm, or more preferably b < 1.5mm. These dimensions enable a very compact structure for the complete probe device, so that efficient deployment e.g. in blood vessels and other regions of the human and/or animal body is possible, e.g. by injecting the probe device to the target region.

According to Applicant's analysis, advantageously, further miniaturization or scaling, respectively, of the magnetic element and the complete probe device comprising said magnetic element is also possible, wherein maximum length dimensions for the magnetic element of e.g. in the micrometer (µm) or even nanometer (nm) range may be attained. This enables to provide probe devices according to the embodiments with outer dimensions of e.g. few hundreds of micrometer or few hundreds of nanometer, thus providing new fields of application.

According to a further embodiment, said magnetic element comprises a remanent magnetic flux density of at least about 0.1 Tesla, T, (1 T = (1 kg)/(A*s²) ), preferably of at least about 1.4 Tesla, which enables to attain high driving torques even for magnetic elements with maximum length dimensions in the micrometer or nanometer range.

As an example, magnetic material of the NdFeB-type, i.e. comprising an alloy of neodymium (Nd), iron (Fe) and boron (B), e.g. Nd2Fe14B, may be used to form the magnetic element or components thereof.

According to some embodiments, the abovementioned bar magnet, which may be considered as a simple exemplary embodiment of a magnetic dipole, is particularly preferred due to its simplicity and commercial availability. However, according to further embodiments, other types of magnetic elements (with non-vanishing magnetic dipole moment), may also be used within the probe device.

According to a further embodiment, the housing of the probe device has a basically ellipsoidal or spherical shape, which facilitates movement or transport, respectively, of said probe device in the target system (e.g. a gastrointestinal (GI) tract or a blood vessel or the like of a human being or an animal). However, according to further embodiments, polygonal or cuboid shapes may also be provided for the housing, especially in cases where it is preferred that the probe device retains its position within the target system.

According to a further embodiment, the magnetic element is rotatably attached to a shaft, wherein said shaft is e.g. fixedly attached to an inner wall of said housing of the probe device or a suitable mechanical support structure provided within said housing. This represents a type of "internal bearing" for the magnetic element.

Alternatively or additionally, an external bearing may be provided for rotatably arranging said magnetic element within said housing of the probe device. For example, according to an embodiment, one or more axial end portions of the magnetic element may be supported or guided within an annular groove provided e.g. on an inner surface of the housing device, which also enables rotatably arranging the magnetic element with respect to the housing. This embodiment does not require an (additional) inner bearing. Other embodiments may provide a fluid (liquid or gas) in an interior section of the housing comprising the magnetic element, wherein an external bearing is formed by the liquid itself.

According to an embodiment, said probe device comprises means for influencing a rotational movement of said magnetic element. This enables the probe device to change e.g. the rotational speed of the magnetic element, which results in a corresponding change of a magnetic field caused by the magnetic element. Such changes of the magnetic field caused by the magnetic element may be registered by an external device (e.g., the external source that may provide an external magnetic field to drive the rotational movement of the magnetic element or any other external device capable of detecting the magnetic field of the probe device's magnetic element).

I.e., generally speaking, according to a further embodiment, said probe device is configured to modulate the rotational movement of its magnetic element (by using said means for influencing a rotational movement of said magnetic element) to transmit data to an external device.

According to a further embodiment, said means for influencing a rotational movement of said magnetic element may comprise at least one of: a) a controllable electric resistor, particularly a controllable switch, connected in parallel to said induction coil, b) a damping element for damping (e.g., directly mechanically, for example by applying friction forces to a shaft of the magnetic element or the magnetic element) rotational movement of said magnetic element, c) an actuator for driving rotational movement of said magnetic element.

According to a particularly preferred embodiment, a controllable switch with two switching states ("on" and "off") may be provided in parallel to the induction coil, wherein the two switching states "on" and "off" enable to choose between applying an open loop or a short circuit to the probe device's induction coil, thus enabling to set two different (inductive) damping factors that correspondingly influence rotational movement of the magnetic element.

According to a further embodiment, said probe device comprises a receiver configured to demodulate said input signal characterizing a voltage induced in said induction coil or a signal derived from said input signal. This enables to use corresponding modulation techniques for transmitting signals to the probe device.

According to a particularly preferred embodiment, an external source providing a magnetic field for the probe device may use frequency modulation, FM, for modulating the magnetic field provided for the probe device. This advantageously enables to provide a continuous signal for transmissions to the probe device, thus attaining optimum energy transfer (as e.g. compared to amplitude modulation, AM, which is characterized by a non-constant signal amplitude and hence time-varying signal power). I.e., while transmitting information to the probe device, a steady energy supply from the external source and the magnetic field provided thereby is ensured.

However, according to further embodiments, other modulation schemes like amplitude modulation or phase modulation may also be used for transmissions to the probe device.

According to a further embodiment, a two-level frequency modulation may be used for data transmissions to the probe device. However, to increase the amount of transferred data per symbol, according to further embodiments, FM schemes with higher number of frequencies can be used, too. Preferably, all frequencies used should be below a "step-out frequency" of the system, i.e. below a critical frequency where the magnetic element of the probe device cannot follow the alternations of the externally applied magnetic field any more. This helps to maintain a continuous rotation of the magnetic element of the probe device.

According to a further embodiment, said probe device comprises a rectifier for rectifying said input signal to obtain a direct current, DC, output voltage, which enables to provide an (externally fed) DC voltage supply within the probe device. The DC voltage may e.g. be used to locally supply the control unit with electric energy.

According to an embodiment, the control unit may e.g. comprise an ASIC (application specific integrated circuit), a microcontroller, a digital signal processor (DSP), discrete logic elements, a programmable logic circuit or any combination thereof. Preferably, said control unit may be optimized for low power supply.

According to further embodiments, the probe device may comprise one or more sensors, which may preferably at least partly be integrated into the housing of the probe device, and which may be configured to measure one or more physical parameters of the probe device and/or a medium surrounding the probe device (i.e., temperature, pressure, pH value).

According to further embodiments, the probe device may comprise one or more actuators, e.g. for controllably releasing agents within a target region, for example in the human body.

According to a further embodiment, said probe device comprises a DC voltage converter to increase the DC output voltage provided by said rectifier, which increases the operational flexibility of the probe device. This way, for example, actuators may be driven which require a comparatively high electric voltage.

Some embodiments feature a method of operating a probe device for use in the human and/or animal body, comprising a housing, at least one magnetic element which is rotatably arranged within said housing, an induction coil that is magnetically coupled with said magnetic element, and a control unit, wherein said control unit processes an input signal characterizing a voltage induced in said induction coil.

According to an embodiment, said probe device influences a rotational movement of said magnetic element. Preferably, said probe device modulates the rotational movement of said magnetic element to transmit data to an external device. This way, sensor data obtained from (optional) sensors integrated in the probe device and/or operational parameters of the probe device and the like may be transmitted to external devices.

Yet further embodiments feature a system comprising at least one probe device according to the embodiments and a transmitter configured to provide a magnetic field around said at least one probe device, wherein said transmitter is further configured to modulate said magnetic field depending on data to be transmitted to said at least one probe device. This enables an efficient data transmission to the probe device, even if the probe device is deployed in a target system such as a human or animal body, a pipe, or the like.

According to an embodiment, said transmitter is configured to use frequency modulation, FM, for modulating said magnetic field, whereby a smooth, steady energy supply to the probe device and at the same time the possibility for efficient data transmission to the probe device is given.

According to a further embodiment, said system comprises a receiver configured to detect a magnetic field. As an example, the receiver may be configured to detect a magnetic field provided by a magnetic element of at least one of said probe devices, which enables data transmissions from the probe device(s) to the system. In the following description, such data transmissions from the probe device(s) to the system or its receiver, respectively, are also denoted as "upstream" data transmissions, while data transmissions from the system or its transmitter, respectively, to the probe device(s) are denoted as "downstream" data transmissions.

According to a further embodiment, said system comprises a set of transmitter coils coupled to said transmitter and a set of receiver coils. The coils are preferably arranged around a target area for the one or more probe devices, e.g. around a human or animal body where the probe device(s) are to be used, so that the transmitter coils may provide a magnetic field around the one or more probe devices, and that the receiver coils may detect the magnetic field(s) caused by the magnetic element(s) of the one or more probe devices.

Preferably, the set of transmitter coils and/or the set of receiver coils comprises at least one pair of Helmholtz coils.

According to a particularly preferred embodiment, said transmitter coils are arranged orthogonally with respect to the receiver coils, which minimizes crosstalk between the upstream and downstream data transmissions effected thereby. According to further embodiments, simultaneous operation of transmitter and receiver coils is also possible.

According to a further embodiment, said transmitter is configured to, in a first operational state, provide an alternating magnetic field of a first amplitude, particularly to drive a rotational movement of the magnetic element of said at least one probe device, wherein optionally, said transmitter is configured to, in a second operational state, provide an alternating magnetic field of a second amplitude, wherein said second amplitude is smaller than said first amplitude. This way, in the first operational state a comparatively strong magnetic field may be generated, which ensures that the magnetic element(s) of all probe devices within the system will be set into rotation.

According to an embodiment, the second amplitude for the second operational state may be zero, i.e. no magnetic field is generated during the second operational state.

According to a further embodiment, a non-vanishing value is chosen for the second amplitude of the second operational state.

According to a further embodiment, said transmitter is configured to assume said first operational state in a first time interval and to assume said second operational state in a second time interval subsequent to said first time interval. According to some embodiments, the second time interval directly follows the first time interval, i.e. there is no delay between these first and second intervals. According to other embodiments, nonzero delays of e.g. some microseconds or even milliseconds are also possible.

According to a further preferred embodiment, said system comprises a plurality of probe devices, wherein a respective device identifier is assigned to each of said probe devices, and wherein said transmitter is configured to address one or more of said probe devices by using their respective device identifier. This way, individual probe devices may be selected to receive downstream data transmissions. I.e., according to an embodiment, a downstream data transmission made by said transmitter may comprise such device identifier of a single probe device to be addressed with said downstream data transmission, and the probe devices receiving said downstream data transmission may evaluate said device identifier. Probe devices having another respective identifier may discard the (further) downstream data transmission addressed to the selected probe device and e.g. assume a passive state in which they particularly do not respond to the downstream data transmission addressed to said selected (other) probe device. In contrast, the "selected" probe device, which has determined that the downstream data transmission comprises its own respective device identifier, may continue to receive and/or evaluate said downstream data transmission. According to an embodiment, if requested by the transmitter, e.g. by means of respective control commands included in such downstream data transmission, said selected (i.e., addressed) probe device may also respond to said downstream data transmission, e.g. in the course of a subsequent upstream data transmission. Such subsequent upstream data transmission from the addressed probe device will usually not be interrupted or influenced in any other way by non-addressed probe devices, as these may assume a passive state as mentioned above.

The above explained embodiments advantageously enable to selectively address single probe devices even in scenarios where a plurality of identical (apart from their respective identifier) or different probe devices is deployed simultaneously in the range of the transmitter.

### Brief description of the figures

Further features, aspects and advantages of the present invention are given in the following detailed description with reference to the drawings in which:
- Figure 1: schematically depicts a block diagram of a probe device according to a first embodiment,
- Figure 2: schematically depicts a block diagram of a probe device according to a second embodiment,
- Figure 3: schematically depicts a block diagram of a probe device according to a third embodiment,
- Figure 4: schematically depicts a block diagram of a control unit of the probe device according to an embodiment,
- Figure 5: schematically depicts a probe device with a basically spherical housing according to a further embodiment,
- Figure 6: schematically depicts a block diagram of a system according to an embodiment,
- Figure 7: schematically depicts a timing of data transmissions to and from a probe device according to an embodiment,
- Figure 8A, 8B, 9: schematically depict signals for downstream data transmissions to a probe device according to an embodiment,
- Figure 10A, 10B: schematically depict signals for upstream data transmissions from a probe device according to an embodiment,
- Figure 11A, 11B: schematically depict signals for upstream data transmissions from a probe device according to a further embodiment,
- Figure 12A, 12B: schematically depict simplified flow-charts of a method of operating a probe device according to further embodiments,
- Figure 13A, 13B: schematically depict simplified flow-charts of a method of operating a system according the embodiments, and
- Figure 14: schematically depicts an operational scenario of a system according to an embodiment with three probe devices deployed in a human body.

### Description of the embodiments

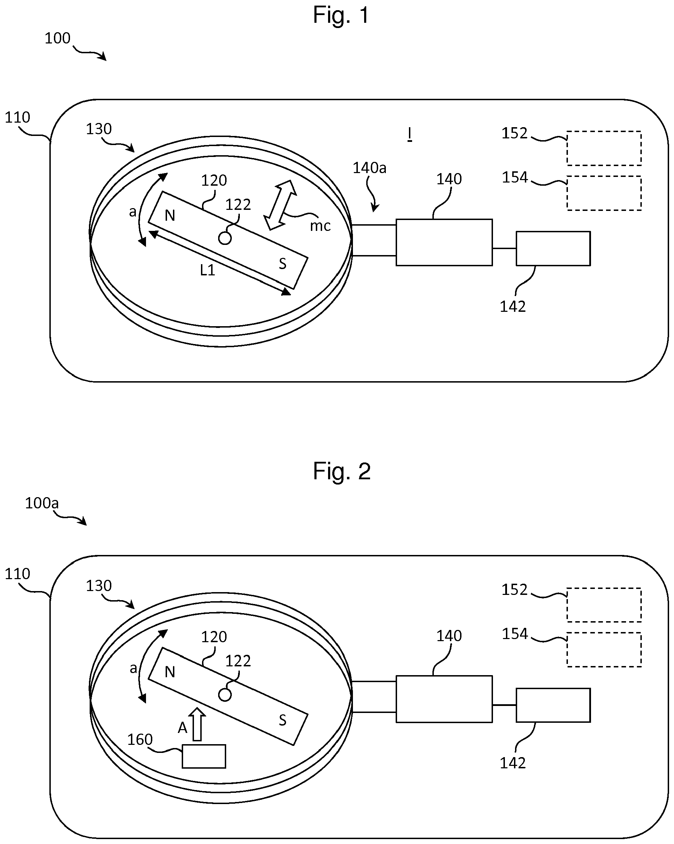

Figure 1 schematically depicts a block diagram of a probe device 100 according to a first embodiment. The probe device 100 is suitable for use e.g. in the human body or in an animal body, e.g. for the purpose of disease management, remote doctor control or personal wellness.

According to the principle of the embodiments, the probe device 100 comprises a housing 110 and at least one magnetic element 120, which is rotatably arranged within said housing 110, so that the magnetic element 120 may rotate basically freely around an axis 122, as illustrated by the double arrow a of Fig. 1.

The housing 110 serves to protect the component(s) in its interior I from influences of a surrounding medium (e.g., blood and/or body tissue), which may be present outside the housing 110.

The configuration of the probe device 100 advantageously enables to supply the probe device 100 with energy from an external source (not shown), e.g. by driving a rotating movement of the magnetic element 120 using magnetic fields generated and/or controlled by said external source. At the same time, exchange of information between the probe device 100 or a component thereof and an external device is enabled, as will be explained in detail further below.

The probe device 100 further comprises an induction coil 130 that is magnetically coupled with said magnetic element 120. This is symbolized in Fig. 1 by the double arrow mc. In the present context, the magnetic coupling between the induction coil 130 and the magnetic element 120 of the probe device 100 means that said induction coil 130 is configured and arranged relatively to said magnetic element 120 such that at least a part of the magnetic flux of the magnetic element 120 may at least temporarily (e.g., depending on an angular position of the rotatably arranged magnetic element 120) pass through said induction coil 130.

Further, the probe device 100 comprises a control unit 140 configured to process an input signal characterizing a voltage induced in said induction coil 130. For this purpose, the control unit 140 is coupled to the induction coil 130 via the lines 140a. The control unit 140 may e.g. comprise an ASIC (application specific integrated circuit), a microcontroller, a digital signal processor (DSP), discrete logic elements, a programmable logic circuit or any combination thereof.

This configuration advantageously enables efficient data exchange between an external device that is capable of generating a magnetic field around the probe device 100 and the probe device 100 without requiring spacious antennas as they are used by conventional systems for transmitting electromagnetic signals in the RF range. Rather, an induction voltage induced into the induction coil 130 by the rotatable magnetic element 120 may be evaluated for receiving information, wherein said magnetic element 120 may be driven by a magnetic field provided for by an external device.

Further advantageously, the probe device 100 does not necessarily require a local storage medium for electric energy such as a battery or the like, as energy may be provided from the external source just in time, e.g. when required, for example when the probe device 100 is deployed in a human body. However, according to other embodiments, a local electric energy storage medium such as a battery and/or a capacitor 142 may be provided, cf. Fig. 1. If capacitors are considered according to some embodiments, double-layer capacitors ("ultracap") are particularly preferred. Presently, the capacitor 142 serves to supply the control unit 140 with electric energy which enables an operation of said control unit 140 even in the absence of external magnetic fields that may be employed for driving said rotational movement of the magnetic element 120 as explained above.

Advantageously, the magnetic fields generated and/or controlled by said external source are not substantially attenuated by the human or animal body so that an efficient energy transfer (and also communication) is enabled between the external source and the probe device 100, even if the probe device 100 is positioned deeply within body tissue.

Although the probe device according to the embodiments is particularly well-suited for use in the human or animal body, according to further embodiments, it may also be used in other environments such as e.g. (biochemical) reactors, pipes, or generally any other target systems where a direct mechanical contact to said probe device is at least temporarily impossible. Also, as the principle according to the embodiments relies on magnetic fields (particularly, low frequency magnetic fields, for example in the range between about 0 Hz and about a few kHz) to drive the rotatable magnetic element 120 of the probe device 100, energy transfer from an external device to said probe device 100 is nearly always possible, except with situations where a strong magnetic shielding (e.g., using material with high magnetic permeability) is applied around the probe device 100, which is usually not the case for applications within human and/or animal bodies.

According to a preferred embodiment, the magnetic element 120 is rotatably attached to a shaft, wherein said shaft is e.g. fixedly attached to an inner wall of said housing 110 (Fig. 1) of the probe device 100 or a suitable mechanical support structure (not shown) provided within said housing 110. This represents a type of "internal bearing" for the magnetic element 120. Presently, a shaft may e.g. be provided at the position of the schematically indicated axis of rotation 122.

Alternatively (or in addition to) an internal bearing, external bearing systems (not shown) may also be used for the magnetic element 120 to enable a rotatable arrangement within the housing 110. For example, one or more axial end portions of said magnetic element 120 may be supported or guided within an annular groove provided e.g. on the inner surface of the housing device.

According to a preferred embodiment, said magnetic element 120 comprises or consists of at least one bar magnet, cf. Fig. 1. For illustration purposes, the magnetic north and south poles "N", "S" of the magnetic element 120 are also indicated in Fig. 1. Preferably, a maximum length dimension L1 of said bar magnet is smaller than about 15 millimeter (mm), preferably smaller than about 1.5 mm, which enables a particularly small configuration. For example, said bar magnet may substantially comprise cuboid shape with a width a, a length b, and a depth c, wherein e.g. b > a and b > c, and wherein said length b e.g. corresponds to the abovementioned maximum length dimension L1, wherein preferably b < 15 mm, or more preferably b < 1.5mm. These dimensions enable a very compact structure for the complete probe device 100, so that efficient deployment e.g. in blood vessels and other regions of the human and/or animal body is possible, e.g. by injecting the probe device 100 to the target region.

Advantageously, according to further embodiments, the housing 110 of the probe device 100 may be designed such that its outer dimension(s) is/are not substantially larger than said maximum length dimension L1 of the magnetic element 120.

According to Applicant's analysis, advantageously, further miniaturization or scaling, respectively, of the magnetic element 120 and the complete probe device 100 comprising said magnetic element 120 is also possible, wherein maximum length dimensions for the magnetic element 120 of e.g. in the micrometer (µm) or even nanometer (nm) range may be attained. This enables to provide probe devices according to the embodiments with outer dimensions of e.g. few hundreds of micrometers or few hundreds of nanometers.

According to a further embodiment, said magnetic element 120 comprises a remanent magnetic flux density of at least about 0.1 Tesla, T, (1 T = (1 kg)/(A*s²) ), preferably of at least about 1.4 Tesla, which enables to attain high driving torques even for magnetic elements 120 with maximum length dimensions L1 in the micrometer or nanometer range.

As an example, magnetic material of the NdFeB-type, i.e. comprising an alloy of neodymium (Nd), iron (Fe) and boron (B), e.g. Nd2Fe14B, may be used to form the magnetic element 120 or components thereof. Permanent magnets made of the above mentioned neodymium alloy may also be denoted as "supermagnets" due to their comparatively high remanent magnetic flux density.

According to some embodiments, the abovementioned bar magnet type having cuboid shape is particularly preferred. However, according to further embodiments, other types of magnetic elements with different shapes may also be used within the probe device 100.

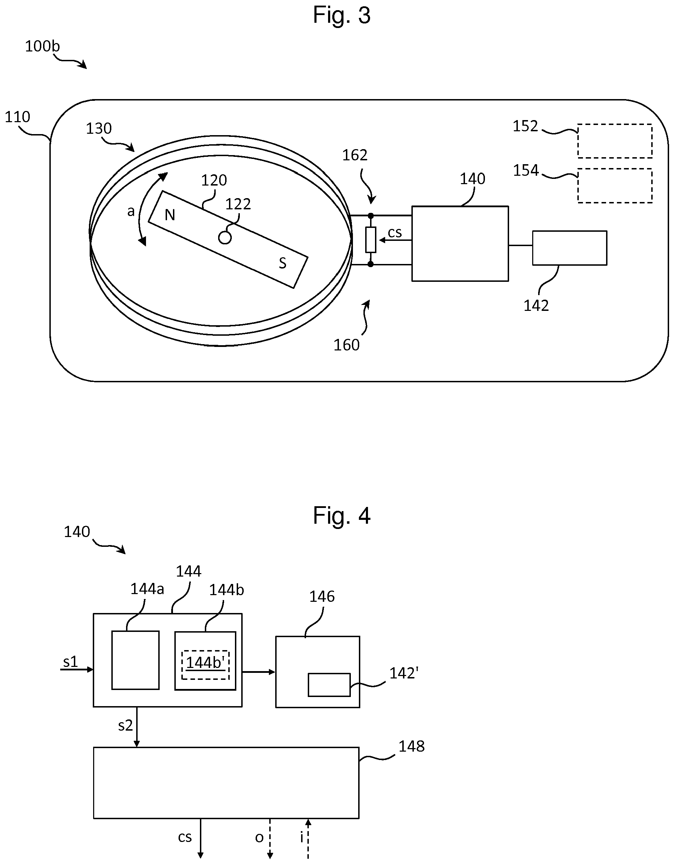

Optionally, according to further embodiments, the probe device 100 may also comprise one or more sensors 152, which may preferably at least partly be integrated into the housing 110 of the probe device 100, and which may be configured to measure one or more physical parameters (i.e. temperature, pressure, pH value, and the like) of the probe device 100 and/or a medium surrounding the probe device 100. In these embodiments, the control unit 140 may evaluate sensor signals provided by said sensors 152. Also, according to further embodiments, the sensor signals or any data derived therefrom may be transmitted from the probe device to an external device, as will be explained in further detail with reference to Fig. 2 and 3 below.

According to further embodiments, the probe device 100 (Fig. 1) may comprise one or more actuators 154, e.g. for controllably releasing agents such as drugs within a target region, for example in the human body, or for manipulating tissue surrounding the probe device 100, or the like.

Advantageously, the operation of the probe device 100 may be remotely controlled by transmitting corresponding control commands to the probe device 100 in form of a modulated external magnetic field, which leads to a correspondingly modulated induction voltage within the induction coil 130 of the probe device 100. The control unit 140 may process this induction voltage as an input signal, e.g. by demodulating and/or filtering and the like, thus obtaining the control commands. For example, such control commands may be used to instruct the probe device 100 to measure physical parameters using the sensor(s) 152, and/or to activate one or more actuators 154, e.g. for releasing a drug into the body.

Figure 2 schematically depicts a block diagram of a probe device 100a according to a further embodiment. Additionally to the components 120, 130, 140 described above with reference to Fig. 1, the probe device 100a of Fig. 2 comprises means 160 for influencing a rotational movement of said magnetic element 120, which is symbolized by the block arrow A in Fig 2. This enables the probe device 100a to change e.g. the rotational speed of the magnetic element 120, which results in a corresponding change of a magnetic field caused or generated by the magnetic element 120. Such changes of the magnetic field caused by the magnetic element 120 may be registered by an external device (e.g., the external source that may provide an external magnetic field to drive the rotational movement of the magnetic element or any other external device capable of detecting the magnetic field of the probe device's magnetic element 120).
According to a further embodiment, said probe device 100a is configured to modulate the rotational movement of its magnetic element 120 (by using said means 160 for influencing a rotational movement of said magnetic element) to transmit data to an external device. The operation of said means 160 may e.g. be controlled by the control unit 140.

According to a further embodiment, said means 160 for influencing a rotational movement of said magnetic element 120 may comprise at least one of: a) a controllable electric resistor, particularly a controllable switch, connected in parallel to said induction coil 130, b) a damping element for damping rotational movement of said magnetic element 120 (e.g., directly mechanically, for example by applying friction forces to a shaft 122 (Fig. 1) of the magnetic element 120 or directly to the magnetic element), c) an actuator (not shown) for driving rotational movement (possibly also increasing rotational speed) of said magnetic element 120.

According to a particularly preferred embodiment, which will be explained in detail below with reference to Fig. 3, a controllable resistor, preferably a switch 162 with two switching states ("on" and "off"), may be provided in parallel to the induction coil 130. Such further embodiment 100b of the probe device equipped with such resistor or switch 162, respectively, is depicted by Fig. 3. The switch may be controlled by the control unit 140, which may output a respective control signal cs to the switch 162. This enables to selectively provide an open loop or a short circuit parallel to the induction coil 130 of the probe device 100b, which corresponds with setting two different (inductive) damping factors that correspondingly influence rotational movement of the magnetic element 120.

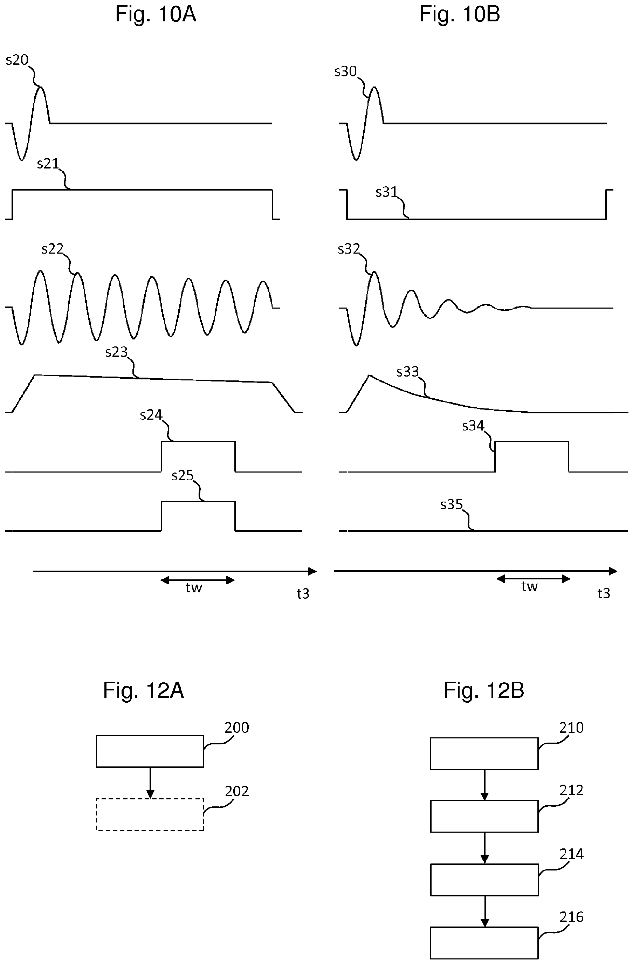

Once the magnetic element 120 of the probe device 100b has been set into rotation (e.g., by application of an external magnetic field), the control unit 140 may selectively apply the abovementioned different damping factors, thus "modulating" a damping of the rotational movement, which corresponds with a variation of the magnetic field generated by the magnetic element 120 that can be measured by an external device having at least one receiver coil for receiving said magnetic field generated by the magnetic element 120. Further details and explanations related to modulating a damping of the rotational movement will be presented further below with reference to Fig. 10A, 10B and Fig. 3.

According to an embodiment, instead of a switch having two switching states, a controllable resistor having more than two controllable resistance values may be used. According to further embodiments, such controllable resistor may e.g. be controlled by applying an analog or a digital control signal cs.

Fig. 4 schematically depicts a block diagram of a control unit 140 of the probe device according to an embodiment.

The control unit 140 comprises a combined receiver and rectifier unit 144 with a receiver 144a, which is configured to demodulate the input signal s1 characterizing a voltage induced in the induction coil 130 of the probe device 100 (Fig. 1), whereby a demodulated signal s2 is obtained. The induced voltage primarily results from the rotational movement of the magnetic element 120. The demodulated signal s2 may be fed to a calculating unit 148, which may control the operation of the probe device 100 accordingly.

For example, the calculating unit 148 may provide the control signal cs for controlling the inductive damping of the magnetic element 120. Further, the calculating unit 148 may provide output signals o to control an operation of the sensor(s) 152 and/or actuators 154. Similarly, the calculating unit 148 may receive input signals i from said sensor(s) 152 and/or actuators 154 (e.g., feedback signals indicating actuation process or the like).

The unit 144 further comprises a rectifier 144b for rectifying said input signal s1 to obtain a direct current, DC, output voltage, which may e.g. be used to charge a capacitor 142' that forms part of an electric energy supply unit 146. The supply unit 146 may e.g. supply the control unit 140 and/or further components of the probe device (e.g., sensors 152 and/or actuators 154) with electric energy.

According to an embodiment, said probe device may comprise a DC voltage converter 144b' (e.g., of the boost converter type) to increase the DC output voltage provided by said rectifier 144b. Presently, as an example, the DC voltage converter 144b' is integrated into the rectifier 144b.

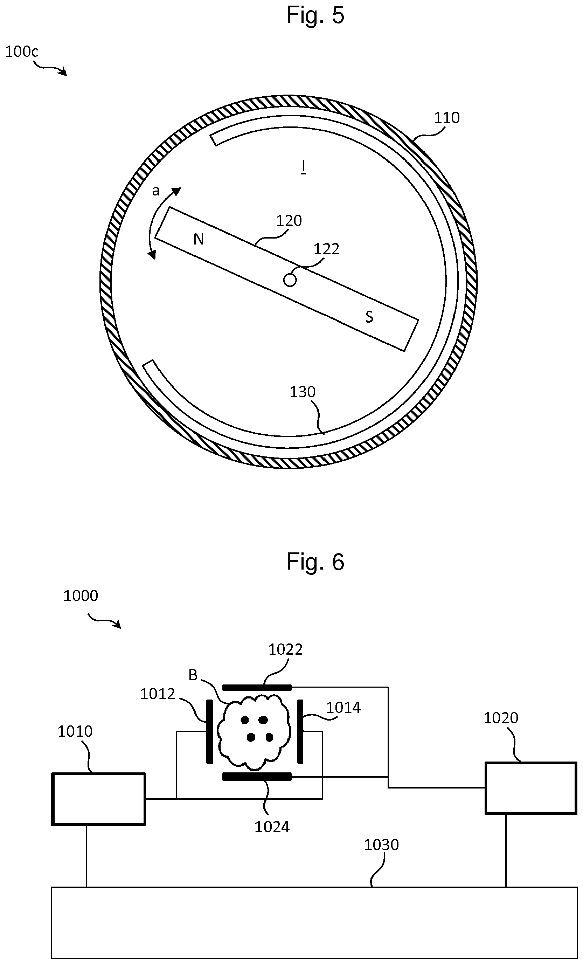

Figure 5 schematically depicts a probe device 100c with a basically spherical housing 110 according to a further embodiment. As can be seen from Fig. 5, the induction coil 130 may e.g. be arranged at an inner surface of the housing 110 thus enabling a strong magnetic coupling between the magnetic element 120 and the induction coil 130.

Preferably, according to some embodiments, the magnetic element 120 and the induction coil 130 (which may also be denoted as "transceiver coil" 130, as it facilitates transmitting and receiving data to/from the probe device) are allocated relative to each other in a way that on one hand the magnetic element 120 can rotate freely "inside" the transceiver coil and on the other hand the moving magnet poles N, S pass near the wires of the transceiver coil 130 in a way that an induction voltage is induced in the transceiver coil 130. According to an embodiment, this induction effect may be optimized by providing substantially right angles between wires of the transceiver coil 130, a direction of movement of the respective magnet poles N, S of the magnetic element 120 and the lines of magnetic flux involved.

Figure 6 schematically depicts a block diagram of a system 1000 according to an embodiment. The system 1000 comprises at least one probe device according to the embodiments. Presently, as an example, four probe devices are depicted being deployed in a human body schematically indicated by reference sign B. Reference signs of the probe devices have been omitted for the sake of clarity. Each of the four depicted probe devices may have a structure as explained above with reference to Fig. 1 to 5, or any combination thereof.

The system 1000 further comprises a transmitter 1010 configured to provide a magnetic field around said at least one probe device, wherein said transmitter 1010 is further configured to modulate said magnetic field depending on data to be transmitted to said at least one probe device.

According to an embodiment, said transmitter 1010 is configured to use frequency modulation, FM, for modulating said magnetic field.

The system 1000 further comprises a receiver 1020 configured to detect a magnetic field, particularly a magnetic field provided by a magnetic element 120 of at least one of said probe devices.

According to a preferred embodiment, the system 1000 comprises a set of transmitter coils 1012, 1014 coupled to said transmitter 1010 and a set of receiver coils 1022, 1024 coupled to the receiver 1020, wherein preferably said transmitter coils 1012, 1014 are arranged orthogonally with respect to the receiver coils 1022, 1024 to minimize crosstalk.

A common control unit 1030 may be provided to control operation of the transmitter 1010 and the receiver 1020. For example, this common control unit 1030 may also be configured for data and/or energy transmission to at least one of the probe device(s) and/or for data reception from at least one of said probe devices.

According to an embodiment, the transmitter 1010, which may also be denoted as "external transmitter", as it is arranged outside of the probe devices and outside of the body B in which the probe devices are deployed, may generate a signal, for example an FM signal, which on the one hand may be demodulated by all probe devices to receive information, and which may, on the other hand, be rectified locally within the probe devices, e.g. as explained with reference to Fig. 4 above, to gain power supply. Similarly, the receiver 1020 of the system 1000 may be denoted as "external receiver" 1020.

According to a further embodiment, if requested from the external transmitter 1010, a control unit 140 of a specific probe device can send information back to the external receiver 1020 by influencing the rotation of its magnetic element 120 (Fig. 1), which can be detected by the external receiver 1020. In other words, according to an embodiment, the system 1000 or its control unit 1030 may be configured to instruct one or more specific probe devices via a (preferably FM modulated) downlink data transmission to perform measurement actions and/or actuator actions and/or upstream data transmissions to the receiver 1020 or the like. Individual probe devices may e.g. be addressed by using a respective device identifier known to both the control unit 1030 and the respective probe device(s).

According to further embodiments, the system 1000 and/or its control unit 1030 may be configured to instruct one or more specific probe devices via a (preferably FM modulated) downlink data transmission to control local actuator(s) the probe device(s) may be equipped with, cf. reference sign 154 of Fig. 1.

Advantageously, the principle according to the embodiments enables to provide a point-to-multipoint communication system 1000 which allows communication between an external transmitter/receiver 1010, 1020, or the common control unit 1030, respectively, and a multitude of probe devices, which may e.g. be used for in-body applications. Further advantageously, the communication system 1000 may also be used to power the probe devices of the system 1000.

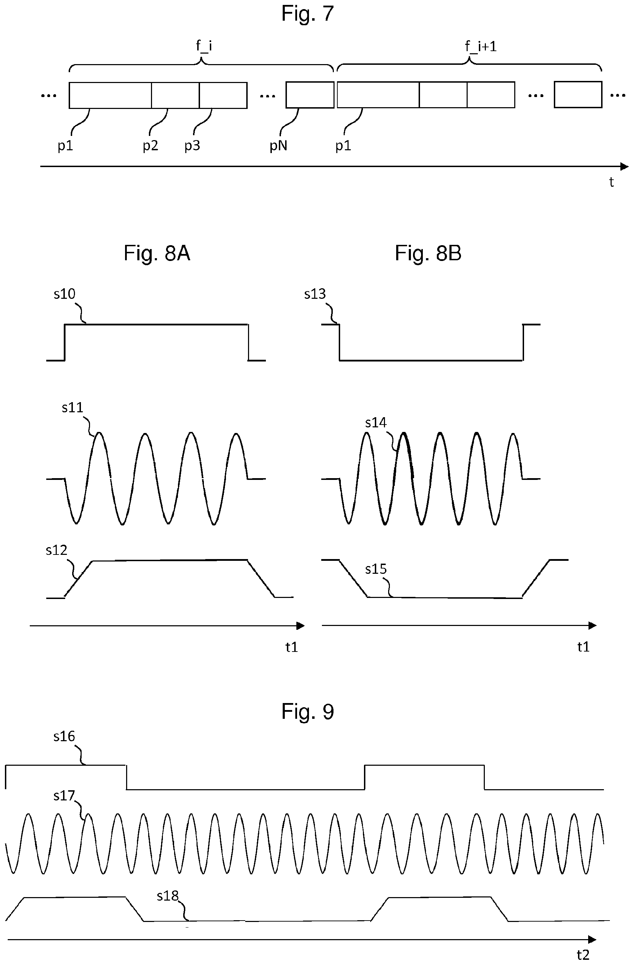

Figure 7 schematically depicts a timing of data transmissions to and from one or more probe device(s) according to an embodiment. Along a time axis t, two subsequent data frames f_i, f_i+1 are depicted.

Each frame consists of a first part p1, which contains downstream data. In this context, a downstream data transmission direction denotes transmissions from the transmitter 1010 of the system 1000, cf. Fig. 6, to the probe device(s). The following parts p2, p3, ..., pN of the frame are used to "poll", or generally, receive, upstream information. In this context, an upstream data transmission direction denotes transmissions from the probe device(s) to the receiver 1020 of the system 1000. Preferably, a signal used for the downstream transmissions p1 may also be used for powering the probe devices.

Figures 8A, 8B, 9 schematically depict signals for downstream data transmissions to a probe device according to an embodiment.

In Fig. 8A, signal s10 exemplarily represents a bit of data to be transmitted in the downstream direction, e.g. from the transmitter 1010 (Fig. 6) to one or more probe devices. The signal s10 uses positive logic, so that the "HIGH" signal level depicted by Fig. 8A represents a logical "1". In contrast, Fig. 8B depicts a case where a logical "0" is to be transmitted in the downstream direction, cf. signal s13.

Returning to Fig. 8A, signal s11 depicts a waveform for an alternating current applied by the transmitter 1010 (Fig. 6) to its transmitter coils 1012, 1014 according to an embodiment. Presently, the signal s11 is a sinusoidal signal having a first frequency f1, which serves to "code" the logical "1" of signal s10. In contrast, signal s14 of Fig. 8B depicts a further waveform for an alternating current applied by the transmitter 1010 (Fig. 6) to its transmitter coils 1012, 1014 according to the present embodiment, the signal s14 representing a sinusoidal signal having a second frequency f2, which is different from the first frequency f1 and which serves to "code" the logical "0" of signal s13. In other words, the signals s11, s14 represent a two-level FM modulation scheme using the first frequency f1 and the second frequency, wherein for example f2 > f1. The further signals s12, s15 represent a demodulated signal for both transmissions cases ("0", "1"), as can e.g. be obtained at an output of the receiver 144a of a probe device according to the embodiments, cf. Fig. 4. E.g., when detecting a magnetic field caused by the current waveform s11 applied to the transmitter coils 1012, 1014, a probe device may detect a received bit "1" after demodulation, whereas when detecting a magnetic field caused by the current waveform s14 applied to the transmitter coils 1012, 1014, a probe device may detect a received bit "0" after demodulation.

The use of FM modulation for downstream transmissions has the further advantage that - simultaneously to the downstream data transmission - an optimum energy transfer to the probe devices may be attained, due to the continuous sinusoidal waveforms. However, according to further embodiments, other modulation schemes like amplitude modulation, AM, or phase modulation (e.g. PSK, phase shift keying) may also be used.

Presently, in the example of Fig. 8A, 8B, for the sake of simplicity, a two-level frequency modulation is used. However, according to further embodiments, to increase the amount of transferred data per symbol, FM schemes with higher numbers of frequencies (e.g., more than two) can be used, too. Preferably, all frequencies used (i.e., f1 and f2 in the present example) shall be below a step-out or critical frequency of the system (more precisely, of the mass system of the magnetic element 120 and its inner/outer bearing), in order to ensure a continuous rotation of the magnetic elements 120 (Fig. 1) in the probe devices following the external magnetic field having the respective frequency f1, f2. If this critical frequency (which is dependent from the amplitude of the externally applied magnet field) is exceeded, a substantial slip characterizing a difference in "rotational speed" of the magnetic field and the rotational speed of the magnetic element 120 may occur (e.g., the magnetic element cannot follow the externally applied magnet field anymore and will eventually stop rotational movement), so that data transmission in the downstream direction may not be possible any more.

Figure 9 exemplarily depicts signals related to a downstream data transmission of the bit sequence "10010" over a time axis t2 according to an embodiment, using the FM scheme explained above with reference to Fig. 8A, 8B. Signal s16 exemplarily represents the logic signal representing said bit sequence "10010", wherein a HIGH level again corresponds with logic "1", and wherein a LO level corresponds with logic "0".

Signal s17 depicts a waveform for an alternating current applied by the transmitter 1010 (Fig. 6) to its transmitter coils 1012, 1014 using subsequent sinusoidal waveform portions of the two different FM frequencies f1, f2 of the present example, wherein the two different FM frequencies f1, f2 of signal s17 characterize the different logic levels of signal s16. Signal s18 represents a demodulated signal as obtained at an output of the receiver 144a (Fig. 4) of a probe device according to the embodiments.

Advantageously, a downstream transmission of the transmitter 1010 (Fig. 6) may be received by all probe devices within its range, e.g. positioned within a region where the magnetic field generated by said transmitter 1010 may be detected. Preferably, said region is substantially defined by a volume between the two transmitter coils 1012, 1014, in which volume the magnetic field is substantially homogenous.

However, according to further embodiments, it may also be sufficient to use one single transmission coil and/or one single receiver coil for the transmitter 1010 and the receiver 1020, respectively.

According to further embodiments, it is also possible to provide more than two coils or even two or more pairs of coils, for the transmitter and/or the receiver.

Figures 10A, 10B schematically depict signals for upstream data transmissions from a probe device to the receiver 1020 (Fig. 6) over a time axis t3 according to an embodiment.

For the following explanations, at first, one single probe device transmitting data in the upstream direction is considered. However, the principle according to the embodiments also enables different probe devices performing individual upstream data transmissions each, preferably following a time-multiplexed manner, wherein e.g. only one probe device is actively transmitting upstream data at a time.

The principle of upstream data transfer of the present embodiment is as follows: In the transmitter coils 1012, 1014 of the transmitter 1010 (Fig. 6), a stimulation signal s20 is generated. Similar to signal s11 explained above with reference to Fig. 8A, the stimulation signal s20 of Fig. 10A may e.g. represent a current applied to the transmitter coils 1012, 1014. The current (amplitude) in the transmitter coils 1012, 1014 is chosen in a way that the magnetic elements 120 (Fig. 1) of preferably all probe devices of the system 1000 (Fig. 6), that are within the range of the transmitter 1010, will be set into rotation (assuming they were in an idle state with no rotation before). Presently, the stimulation signal s20 exemplarily comprises one single period of a sinusoidal waveform.

If the stimulation signal s20 is switched off again after said single period, the further rotational movement behavior of each magnetic element 120 (Fig. 1) may be influenced by the damping techniques explained above with reference to Figures 2, 3. For example, it is assumed that the probe devices presently involved comprise a controllable switch 162 as depicted by Fig. 3, enabling them to selectively damp or not damp the rotational movement of the magnetic element 120, which has been initiated by application of the stimulation signal s20.

For example, if the switch 162 is closed under control of the control signal cs issued by the local control unit 140 of the probe device 100b (Fig. 3), the rotation of the magnetic element 120 will be significantly damped (and possibly even stopped after few rotations), because in this case the induction coil 130 is short-circuited by said switch 162. On the other hand, if the switch 162 is open, the rotation of the magnetic element 120 triggered by the stimulation signal s20 will continue for a significant longer period of time, as compared to the case with the switch 162 being closed.

The rotation of the magnetic element 120 of the "transmitting" probe device 100b is detected by the external receiver 1020 (Fig. 6) connected to the external receiver coils 1022, 1024.

For example, if a logic "1" is to be transmitted by the probe device in the upstream direction, which is indicated by signal s21 of Fig. 10A having a HIGH level, the probe device controls its switch 162 to the open state, thus not actively damping the rotational movement of its magnetic element 120 that has been started by the stimulation signal s20. Hence, the resulting "receive" signal induced into the receiver coils 1022, 1024 by the substantially freely rotating magnetic element 120 of the transmitting probe device may comprise the shape as depicted by signal s22 of Fig. 10A, i.e. a slightly damped periodic signal, said slight damping e.g. resulting from friction losses and other parasitic effects within the transmitting probe device.

In contrast, if a logic "0" is to be transmitted by the probe device in the upstream direction, which is indicated by signal s31 of Fig. 10B having a LO level, the probe device controls its switch 162 to the closed state, thus actively damping the rotational movement of its magnetic element 120 that has been started by the stimulation signal s30, which is also depicted by Fig. 10B. Hence, the resulting "receive" signal induced into the receiver coils 1022, 1024 by the actively damped magnetic element 120 of the transmitting probe device may comprise the shape as depicted by signal s32 of Fig. 10B, i.e. a strongly damped periodic signal, said strong damping e.g. resulting from a superposition of the above described active damping by means of switch 162 and friction losses and other parasitic effects within the transmitting probe device.

For both cases, detected signals that may e.g. be obtained from rectifying the signals s22, s32 ("envelope detection") are represented by signal s23 of Fig. 10A and signal s33 of Fig. 10B.

According to a preferred embodiment, for further evaluating the detected signals s23, s33, e.g. within the control unit 1030 of the system 1000 of Fig. 6, a gating signal s24, s34 may be used, which defines a time window tw within which the detected signals s23, s33 are to be evaluated.

Preferably, the gating signals s24, s34 and the resulting time windows are identical for both receiving cases "1" (Fig. 10A) and "0" (Fig. 10B).

According to an embodiment, the start of the time window tw is chosen with respect to the start or end of the stimulating signal s20, s30 such that for the different receive cases "1"/"0" a clear distinction between the respective signal levels of the detected signals s23, s33 can be made, which is enabled by the different damping characteristics of said both cases.

As within the time window tw the detected signal s23 of the "1" case is above a predetermined threshold used for distinguishing between "1" and "0" detected signals, signal s25, which is a gated version of said detected signal s23, indicates that a "1" has been received from the probe device. In contrast, as within the time window tw the detected signal s33 of the "0" case is below said predetermined threshold used for distinguishing between "1" and "0" detected signals, signal s35, which is a gated version of said detected signal s33, indicates that a "0" has been received from the probe device.

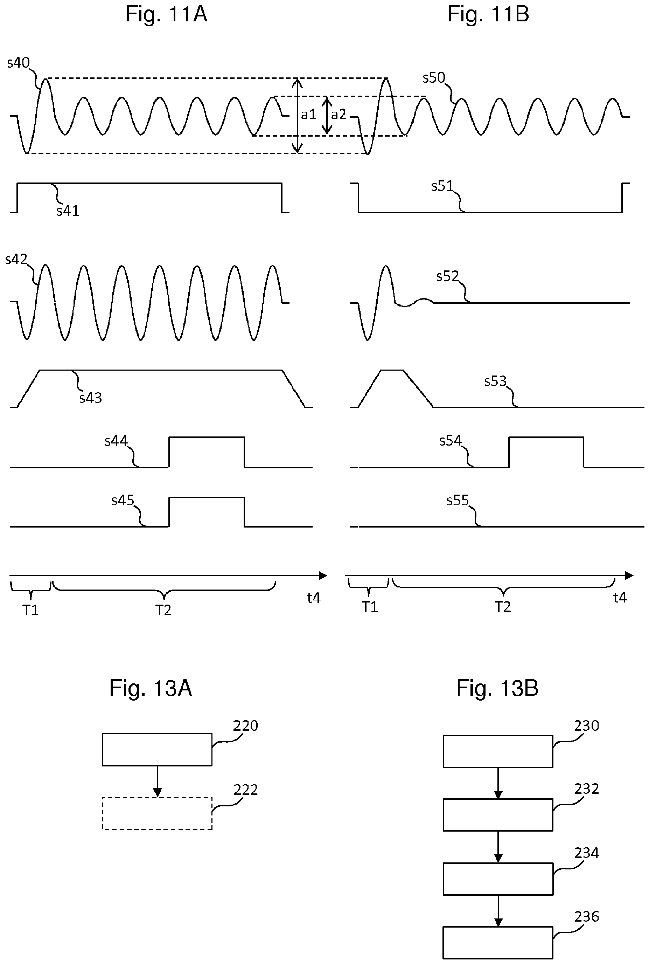

Figures 11A, 11B schematically depict signals for upstream data transmissions from a probe device to the receiver 1020 (Fig. 6) over a time axis t4 according to a further embodiment.

The principle of upstream data transfer of the present embodiment is as follows: Similar to the embodiment of Figures 10A, 10B, in the external transmitter coils 1022, 1024 (Fig.6), a stimulation signal s40, s50 is generated. For example, the stimulation signals s40, s50 may e.g. represent a respective current applied to the transmitter coils 1012, 1014.

As with the preceding embodiments, Fig. 11A represents a case where a "1" is to be sent from the probe device, whereas Fig. 11B represents a case where a "0" is to be sent from the probe device. For both cases, the stimulation signals s40, s50 are identical according to the present embodiment.

The signal s41 of Fig. 11A indicates by its HIGH level that a "1" is to be transmitted, while the signal s51 of Fig. 11B indicates by its LO level that a "0" is to be transmitted. In other words, if a logic "1" is to be transmitted by the probe device in the upstream direction, this is indicated by signal s41 of Fig. 11A having a HIGH level, and if a logic "0" is to be transmitted by the probe device in the upstream direction, this is indicated by signal s51 of Fig. 11B having a LO level, similar to signals s21, s31 explained above with reference to the embodiments of Fig. 10A, 10B.

Similar to the preceding embodiment of Figures 10A, 10B, the stimulation signals s40, s50 of Figures 11A, 11B have basically sinusoidal shape with a certain frequency the rotating magnetic elements 120 of the probe devices can follow. However, in difference to the preceding embodiment of Figures 10A, 10B, the stimulation signals s40, s50 of Figures 11A, 11B have two different amplitude levels a1, a2.

I.e., in the present embodiment, the transmitter 1010 (Fig. 6) is configured to, in a first operational state, provide an alternating magnetic field of a first amplitude, particularly to drive a rotational movement of the magnetic element 120 of said at least one probe device. Optionally, said transmitter 1010 is further configured to, in a second operational state, provide an alternating magnetic field of a second amplitude, wherein said second amplitude is smaller than said first amplitude. This is achieved by using the first amplitude a1 (Fig. 11A) within the first time interval T1 of signal s40 of Fig. 11A, and by using the second amplitude a2 < a1 within the second time interval T2 of signal s40 of Fig. 11A, which time intervals correspond with said first and second operational states. The same principle is also used for the stimulation signal s50 of Figure 11B.

According to a preferred embodiment, the first current level a1 is chosen in a way that the magnetic elements 120 in all involved probe devices will be set into rotation.

The second current level a2 is chosen in the way that the magnetic elements 120 of such probe devices 100b (Fig. 3) which have an open controllable switch 162 (i.e., no active damping of the induction coil 130, see Fig. 3) will continue rotating under application of said second current level a2. This is indicated by a "receive" signal s42 induced into the receiver coils 1022, 1024 by the substantially freely rotating magnetic element 120 of the transmitting probe device. As can be seen, expectedly, the amplitude of the signal s42 remains constant during both time intervals T1, T2.

However, according to an embodiment, the behavior of a magnetic element 120 in a probe device which has a closed switch 162 (i.e., active damping of the rotating magnetic element 120 by means of the short-circuited induction coil 130 enabled, this case being represented by Fig. 11B) will be significantly different: the closed switch 162 will damp the magnetic element 120 in a way that the step-out (or critical) frequency of the system (for a given amplitude of the externally applied magnetic field) will be exceeded, and as a consequence the magnetic element of such probe device can no longer follow the magnetic field characterized by signal s40, s50 with the second amplitude a2 level, and rotation of the magnetic element will stop more or less immediately. This is indicated by a "receive" signal s52 induced into the receiver coils 1022, 1024 by the actively damped rotating magnetic element 120 of the respective transmitting probe device. As can be seen, expectedly, the amplitude of the signal s52 quickly approaches zero after the beginning of the second time interval T2. Generally, it is to be noted that the abovementioned critical frequency of the system depends on the amplitude of the externally applied magnetic field. I.e., it is expected that the critical frequency increases with larger amplitudes of the externally applied magnetic field.

For both cases, detected signals that may e.g. be obtained from rectifying the signals s42, s52 ("envelope detection") are represented by signal s43 of Fig. 11A and signal s53 of Fig. 11B.

According to a preferred embodiment, for further evaluating the detected signals s43, s53, e.g. within the control unit 1030 of the system 1000 of Fig. 6, a gating signal s44, s54 may be used, which defines a time window (also cf. Fig. 10A, 10B) within which the detected signals s43, s53 are to be evaluated. Preferably, the gating signals s44, s54 and the resulting time windows are identical for both receiving cases "1" (Fig. 11A) and "0" (Fig. 11B).

Similar to the preceding embodiment of Fig. 10A, 10B, the start of the time window for the gating signals s44, s54 of Fig. 11A, 11B is chosen with respect to the start or end of the first time interval T1 of the stimulating signal s40, s50 such that for the different receive cases "1"/"0" a clear distinction between the respective signal levels of the detected signals s43, s53 can be made, which is enabled by the significantly different damping characteristics of said both cases.

However, in difference to the preceding embodiment of Fig. 10A, 10B, for the current embodiment of Fig. 11A, 11B, the difference between signal values of the detected signals s43, s53 for both receive cases "1", "0" is even more significant, because the stronger damping of signal s52 caused by the presently used specific stimulation signals s40, s50 usually leads to a signal value of zero for the detected signal s53 in the relevant time window represented by gating signal s54.

Consequently, signal s45, which is a gated version of said detected signal s43, indicates that a "1" has been received from the probe device. In contrast, signal s55, which is a gated version of said detected signal s53, indicates that a "0" has been received from the probe device.

According to a preferred embodiment, the received signals s42, s52 induced in the receiver coils 1022, 1024 are continuous for an open controllable switch 162 (Fig. 3). Further, the received signals s42, s52 induced in the receiver coils 1022, 1024 are advantageously phase-locked to the respective stimulation signals s40, s50. According to an embodiment, this can favorably be exploited for e.g. synchronous detection of the receiver signal, e.g. within the control unit 1030.

Figure 12A schematically depicts a simplified flow-chart of a method of operating a probe device 100 according to the embodiments. In step 200, the control unit 140 (Fig. 1) of the probe device processes an input signal s1 (cf. Fig. 4) characterizing a voltage induced in the induction coil 130 of the probe device 100, wherein said induced voltage may be caused by a magnetic field applied to the probe device 100, e.g. by means of a transmitter 1010 as depicted by Fig. 6 (said applied magnetic field driving the magnetic element 120, which, in turn, induces said voltage into the induction coil 130). Said processing may e.g. comprise demodulating said input signal s1, e.g. by rectifying said input signal s1. Optionally, a step of low-pass filtering may follow upon the step of rectifying. A so demodulated signal s2, also cf. Fig. 4, may be fed to a calculating unit 148, which may control the operation of the probe device 100 accordingly. This is symbolized by optional step 202 of Fig. 12A.

Advantageously, while receiving an input signal s1, at least a portion of this input signal s1 may also be used for electric energy supply of the probe device, e.g. by rectifying the input signal s1 and charging a local capacitor or the like.

According to some embodiments, in step 202, sensor data from one or more sensors 152 (Fig. 1) integrated to the probe device or associated with the probe device may be retrieved by the control unit 140 of the probe device.

Also, transmission of data in an upstream direction, e.g. to the "external" receiver 1020 (Fig. 6), may be performed in step 202. This may e.g. be achieved by influencing a rotational movement of the magnetic element 120 of the probe device, e.g. once this magnetic element 120 has been set into rotation by application of an appropriate stimulating external magnetic field, for example by means of the transmitter 1010 (Fig. 6). Particularly, a specific modulation of the rotational movement of the magnetic element 120 of the probe device may be applied in step 202 (Fig. 12A), e.g. by selectively opening and/or closing the controllable switch 162 (Fig. 3) or any other means 160 (Fig. 2) for influencing a rotational movement of the magnetic element 120.

Figure 12B schematically depicts a simplified flow-chart of a method of operating a probe device 100b (Fig. 3) according to a further embodiment. In step 210, the probe device 100b receives energy from an externally generated magnetic field, which is generated by the transmitter 1010 (Fig. 6) of the system 1000, and charges a local capacitor.

In step 212 (which may also occur simultaneously to step 210), a downstream data transmission from the transmitter 1010 commanded by the control unit 1030 is received by the probe device 100b, cf. signal portion p1 of the frame f_i of Fig. 7. This data transmission may e.g. comprise instructions for the probe device 100b to transmit in an upstream direction data to the receiver 1020, wherein said instructions may be evaluated by the probe device 100b. Such downstream data transmission may e.g. be performed according to the embodiments explained above with reference to Figures 8A to 9.

In step 214 (Fig. 12B), the probe device 100b performs the instructed upstream data transmission, e.g. in the upstream time slot indicated by reference sign p3 of the frame f_i of Fig. 7. Such upstream data transmission may e.g. be performed according to the embodiments explained above with reference to Figures 10A to 11B.

In step 216, the probe device 100b waits for a next data frame f_i+1 expected to be transmitted by the transmitter 1010.

The sequence of steps 210, 212, 214, 216 explained above with reference to Fig. 12B is provided for illustration purposes. According to further embodiments, one or more steps may also be omitted or may have another sequence relative to each other.

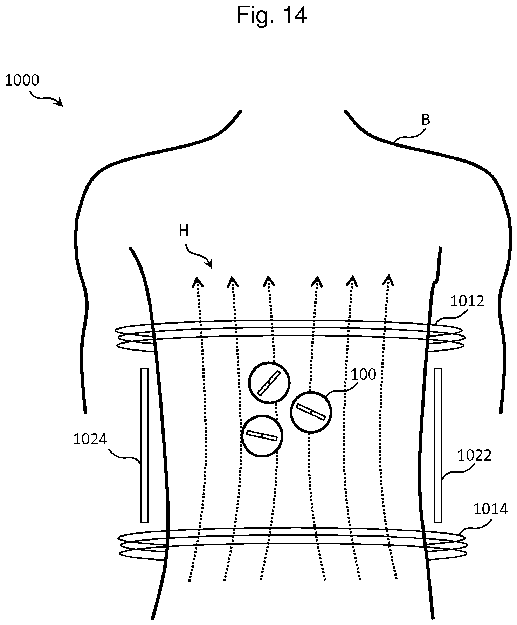

Figure 13A schematically depicts a simplified flow-chart of a method of operating a system 1000 according to an embodiment. For this purpose, reference is also made to Figure 14, which schematically depicts an operational scenario of the system 1000 according to an embodiment with three probe devices 100 deployed in a human body B.

In a first step 220, the transmitter 1010 (Fig. 6) of the system 1000 provides a magnetic field H, cf. Fig. 14, within a region of the human body B that comprises the probe devices 100. For example, the probe devices 100 are positioned within a gastrointestinal, GI, tract of the body B, for analyzing local physical parameters such as a pH value and temperature of tissue thereof.

Providing 220 said magnetic field H may comprise providing an alternating magnetic field H, e.g. of basically sinusoidal waveform, which may be effected by applying corresponding electrical currents to the transmitter coils 1012, 1014. Note that the transmitter 1010 / receiver 1020 of the system 1000 as well as electrical connections between the coils 1012, 1014, 1022, 1024 and the transmitter 1010 / receiver 1020 of the system 1000 are not depicted by Fig. 14 for the sake of clarity. However, the corresponding topology is e.g. depicted by Fig. 6.

For example, according to an embodiment, electric currents with waveforms corresponding to at least one of the signals s11, s14, s17, S20, s30, s40, s40 explained above with reference to Fig. 8A, 8B, 9, 10A, 10B, 11A, 11B may be applied to the transmitter coils 1012, 1014 in step 220.

Optionally, in step 222 (Fig. 13A), the magnetic field H may be modulated, e.g. by using FM modulation according to the embodiments explained above with reference to Fig. 8A, 8B, 9. For example, according to an embodiment, electric currents with a waveform corresponding to the signal s17 of Fig. 9 may be applied to the transmitter coils 1012, 1014 in step 222 for effecting said modulation, e.g. for the purpose of downstream data transmission to the probe devices 100.

Figure 13B schematically depicts a simplified flow-chart of a method of operating a system 1000 according to a further embodiment.

In step 230, transmitter 1010 performs a downstream data transmission to the probe devices 100 comprised within its range, i.e. the probe devices 100 arranged in the GI tract of the body B. Said data transmission may comprise in a first part p1 of a data frame f_i (Fig. 7) scheduling information characterizing individual ones of said the probe devices 100 and associated upstream transmission resources of a data frame, possibly the same data frame f_i.

For example, the scheduling information may comprise tuples comprising an identifier of a specific probe device 100 and an associated upstream resource such as a specific further data portion p2, p3 of the frame f_i the specific probe device 100 may use for its next upstream transmission. E.g., said scheduling information may indicate that a specific probe device 100 may use the data portion p2 of the instant data frame f_i (and optionally also another data portion p3 of the next data frame f_i+1) for its next upstream transmission(s).

In step 232, said specific (first) probe device transmits data in the upstream direction, e.g. using data portion p2 of the frame f_i (Fig. 7), which data is received by the receiver 1020 (Fig. 6) in the same step 232.

In a next step 234, a second one of said probe devices transmits data in the upstream direction, e.g. using data portion p3 of the frame f_i (Fig. 7), which data is received by the receiver 1020 (Fig. 6) in said step 234.

In subsequent step 236, the receiver 1020 receives data from further probe devices.

According to a further embodiment, if multiple probe devices 100 (Fig. 14) are present within an operating area or effective range, respectively, of the transmitter 1010 of the system 1000 (Fig. 6), all probe devices 100 may receive downstream "broadcast" data transmitted by the transmitter 1010.

According to a further embodiment, in the upstream direction, preferably one probe device should be active in a given part p2, p3 (Fig. 7) of a given frame f_i, f_i+1. This may e.g. be achieved in the way that all probe devices 100 keep their related switch 162 (Fig. 3) closed during upstream part p2, p3, ..., pN of the frames f_i, f_i+1, .., and only the specific probe device which is allowed to transmit in a given upstream data field (e.g., as indicated by a "scheduling grant" of a preceding downstream transmission) will open and close, i.e. modulate, its switch 162 in this upstream data field.

According to further embodiments, an allocation of a given upstream data field to a certain probe device can be done following conventional multiple access principles. According to a further embodiment, this e.g. includes statically fixed allocation of time slots, but can also comprise transmission of a list of devices in downstream part of frame which allows those devices to send in respective allocated upstream data fields, either of the same frame or one of the following frames.

While bidirectional communication between the control unit 1030 and the probe device(s) represents a preferred aspect, according to further embodiments it is also possible that the system (only) comprises one or more transmitter coils for transmitting signals in a downstream direction to the probe device(s), which may still enable to control an operation of the probe device(s) and to supply them with energy.

According to a further embodiment, one or more magnetic coils may be provided for use with both the transmitter 1010 and the receiver 1020, preferably in a time-multiplexed manner. E.g., during a first time interval, the coil(s) may be used for downstream transmissions and/or energy supply to the probe device(s), wherein in a second time interval, which is different from said first time interval, said same coil(s) may be used for receiving upstream transmissions from said probe device(s).

The probe devices according to the embodiments may advantageously be used within a human or animal body, e.g., for measurement or drug delivery purposes or the like.

According to Applicant's analysis, advantageously, miniaturization or scaling, respectively, of the magnetic element 120 and the complete probe device 100, 100a, 100b, 100c comprising said magnetic element 120 is possible, wherein maximum length dimensions for the magnetic element 120 of e.g. in the micrometer (µm) or even nanometer (nm) range may be attained. This enables to provide probe devices according to the embodiments with outer dimensions of e.g. few hundreds of micrometer or few hundreds of nanometer, thus providing new fields of application.

Further advantageously, the principle according to the embodiments enables to provide a scalable communication system which on one hand can cope with the decreasing size of the probe devices (from centimeter scale via millimeter to micrometer or even nanometer scale) and on the other hand is able to establish individual communication to a multitude of such devices 100 e.g. deployed in the human body B. Particularly, an efficient point-to-multipoint communication from the transmitter 1010 (Fig. 6) to multiple probe devices 100 is enabled, wherein additionally energy supply for the probe devices 100 is enabled simultaneously to the communication.

As a further advantage, the principle of the embodiments does not require a local energy storage such as a battery within the probe devices, as energy required for operation may be delivered just when it is needed, e.g. in form of an externally applied magnetic field H. This way, the operating time of the probe device according to the embodiments is basically not limited by such local energy storage. However, according to some embodiments, a local buffer such as a capacitor may be beneficial to facilitate stabilizing a supply voltage for components 140, 152, 154 (Fig. 1) of the probe device.

As a further aspect, the principle according to the embodiments enables communication from outside a body B with a multitude of probe devices 100 inside this body B ("in-body devices"), cf. Fig. 14. By exploiting rotating magnetic fields according to an embodiment, a size of the in-body devices 100 is no longer dependent from e.g. dimensions required for conventional RF antennas, so the size of the devices 100 can follow miniaturization requirements, which miniaturization is advantageously enabled applying the principle according to the embodiments. Furthermore, according to other embodiments, integrated energy transport to in-body devices 100 allows virtually unlimited lifetime of such devices (in contrast to usage of built-in batteries within conventional probes, which always have a limited livetime).

The description and drawings merely illustrate the principles of the invention. It will thus be appreciated that those skilled in the art will be able to devise various arrangements that, although not explicitly described or shown herein, embody the principles of the invention and are included within its spirit and scope. Furthermore, all examples recited herein are principally intended expressly to be only for pedagogical purposes to aid the reader in understanding the principles of the invention and the concepts contributed by the inventor(s) to furthering the art, and are to be construed as being without limitation to such specifically recited examples and conditions. Moreover, all statements herein reciting principles, aspects, and embodiments of the invention, as well as specific examples thereof, are intended to encompass equivalents thereof.

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

A person of skill in the art would readily recognize that steps of various above-described methods can be performed by programmed computers. Herein, some embodiments are also intended to cover program storage devices, e.g., digital data storage media, which are machine or computer readable and encode machine-executable or computer-executable programs of instructions, wherein said instructions perform some or all of the steps of said above-described methods. The program storage devices may be, e.g., digital memories, magnetic storage media such as a magnetic disks and magnetic tapes, hard drives, or optically readable digital data storage media. The embodiments are also intended to cover computers programmed to perform said steps of the above-described methods.

The functions of the various elements shown in the FIGS., including any functional blocks labeled as "processors", may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" or "controller" should not be construed to refer exclusively to hardware capable of executing software, and may implicitly include, without limitation, digital signal processor (DSP) hardware, network processor, application specific integrated circuit (ASIC), field programmable gate array (FPGA), read only memory (ROM) for storing software, random access memory (RAM), and non-volatile storage. Other hardware, conventional and/or custom, may also be included. Similarly, any switches shown in the FIGS. are conceptual only. Their function may be carried out through the operation of program logic, through dedicated logic, through the interaction of program control and dedicated logic, or even manually, the particular technique being selectable by the implementer as more specifically understood from the context.

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

## Claims

1. Probe device (100; 100a; 100b; 100c) for use in the human and/or animal body, comprising a housing (110), at least one magnetic element (120) which is rotatably arranged within said housing (110), an induction coil (130) that is magnetically coupled with said magnetic element (120), and a control unit (140) configured to process an input signal (s1) characterizing a voltage induced in said induction coil (130).

2. Probe device (100; 100a; 100b; 100c) according to claim 1, wherein said probe device (100; 100a; 100b; 100c) comprises means (160) for influencing a rotational movement of said magnetic element (120).

3. Probe device (100; 100a; 100b; 100c) according to claim 2, wherein said probe device (100; 100a; 100b; 100c) is configured to modulate the rotational movement of said magnetic element (120) to transmit data to an external device (1020).

4. Probe device (100; 100a; 100b; 100c) according to one of the claims 2 to 3, wherein said means (160) for influencing a rotational movement of said magnetic element (120) comprise at least one of: a) a controllable electric resistor (162), particularly a controllable switch, connected in parallel to said induction coil (130), b) a damping element for damping rotational movement of said magnetic element (120), c) an actuator for driving rotational movement of said magnetic element (120).

5. Probe device (100; 100a; 100b; 100c) according to one of the preceding claims, wherein said probe device comprises a receiver (144a) configured to demodulate said input signal (s1) or a signal derived from said input signal (s1).

6. Probe device (100; 100a; 100b; 100c) according to one of the preceding claims, wherein said probe device comprises a rectifier (144b) for rectifying said input signal (s1) to obtain a direct current, DC, output voltage.

7. Probe device (100; 100a; 100b; 100c) according to claim 6, wherein said probe device (100; 100a; 100b; 100c) comprises a DC voltage converter to increase the DC output voltage provided by said rectifier (144b).

8. Method of operating a probe device (100; 100a; 100b; 100c) for use in the human and/or animal body, comprising a housing (110), at least one magnetic element (120) which is rotatably arranged within said housing (110), an induction coil (130) that is magnetically coupled with said magnetic element (120), and a control unit (140), wherein said control unit (140) processes (200) an input signal (s1) characterizing a voltage induced in said induction coil (130).

9. Method according to claim 8, wherein said probe device (100; 100a; 100b; 100c) influences a rotational movement of said magnetic element (120).

10. System (1000) comprising at least one probe device (100; 100a; 100b; 100c) according to one of the claims 1 to 7 and a transmitter (1010) configured to provide (220) a magnetic field (H) around said at least one probe device (100; 100a; 100b; 100c), wherein said transmitter (1010) is further configured to modulate (222) said magnetic field (H) depending on data to be transmitted to said at least one probe device (100; 100a; 100b; 100c).

11. System (1000) according to claim 10, wherein said transmitter (1010) is configured to use frequency modulation, FM, for modulating (222) said magnetic field (H).

12. System (1000) according to one of the claims 10 to 11, wherein said system (1000) comprises a receiver (1020) configured to detect a magnetic field.

13. System (1000) according to one of the claims 10 to 12, wherein said system (1000) comprises a set of transmitter coils (1012, 1014) coupled to said transmitter (1010) and a set of receiver coils (1022, 1024).

14. System (1000) according to one of the claims 10 to 13, wherein said transmitter (1010) is configured to, in a first operational state, provide an alternating magnetic field (H) of a first amplitude.

15. System (1000) according to claim 14, wherein said transmitter (1010) is configured to assume said first operational state in a first time interval (T1) and to assume a second operational state in a second time interval (T2) subsequent to said first time interval (T1).

16. System (1000) according to one of the claims 10 to 15, wherein said system (1000) comprises a plurality of probe devices (100; 100a; 100b; 100c) according to one of the claims 1 to 7, wherein a respective device identifier is assigned to each of said probe devices (100; 100a; 100b; 100c), and wherein said transmitter (1010) is configured to address one or more of said probe devices (100; 100a; 100b; 100c) by using their respective device identifier.
